# EUROPÄISCHE PATENTSCHRIFT

(11) **EP 2 591 480 B1**
(45) Veröffentlichungstag und Bekanntmachung des Hinweises auf die Patenterteilung: **01.08.2018**
(21) Anmeldenummer: 11714937.7
(22) Anmeldetag: 06.04.2011
(51) Int. Cl.: H01F 7/122, H01F 7/16

(54) **ELEKTROMAGNETISCHER AKTUATOR FÜR EIN CHIRURGISCHES INSTRUMENT**
ELECTROMAGNETIC ACTUATOR FOR A SURGICAL INSTRUMENT
ACTIONNEUR ÉLECTROMAGNÉTIQUE POUR INSTRUMENT CHIRURGICAL

(30) Priorität: 05.07.2010 DE 102010030919
(43) Veröffentlichungstag der Anmeldung: 15.05.2013
(73) Patentinhaber: Olympus Winter&Ibe GmbH, 22045 Hamburg (DE)
(72) Erfinder: WIETERS, Martin, 22885 Barsbüttel (DE); FELDMANN, Marco, 22085 Hamburg (DE)
(74) Vertreter: Seemann & Partner Patentanwälte mbB
(86) Internationale Anmeldenummer: PCT/EP2011/001699
(87) Internationale Veröffentlichungsnummer: WO 2012/003897

(56) Entgegenhaltungen:
- EP-A2- 2 175 458
- DE-A1- 3 717 872
- DE-B- 1 253 407
- DE-U1- 20 000 397

## Beschreibung

Die Erfindung betrifft einen elektromagnetischen Aktuator für ein chirurgisches oder medizinisches Instrument, insbesondere Endoskop, wobei der Aktuator einen Stator und ein verschiebbares Element aufweist, das wenigstens teilweise ein paramagnetisches und/oder ferromagnetisches Material aufweist und durch Beaufschlagung mit einem elektromagnetischen Feld von einer ersten Position in eine zweite Position verschiebbar ist.

Aus DE 196 18 355 C2 ist ein Endoskop mit einem distal angeordneten Objektiv bekannt, dessen Bild ein Bildweiterleiter zum proximalen Ende weiterleitet und das wenigstens ein optisches Element wie eine Linsengruppe aufweist, das in Richtung der optischen Achse zur Fokussierung und/oder zur Veränderung der Brennweite durch einen Mikroantrieb verschiebbar ist, wobei der Mikroantrieb wenigstens eine rotationssymmetrische axial bewegliche Hülse aufweist, die die Linsen bzw. das optische Element der bewegbaren Linsengruppe umgibt und aufnimmt, und wobei die Hülse aus einem permanent magnetischen Material besteht, und in einem Magnetfeld beweglich ist, das von einer Spulenanordnung erzeugt wird. Um die Hülse zu bewegen und zu halten, wird dauernd ein elektromagnetisches Feld erzeugt.

Aus DE 1 253 407 B ist ein Endoskop mit einer distal ausstrahlenden Beleuchtungseinrichtung für einen zu beobachtenden Körperhöhlenteil und einem Bildleiter bekannt, von dem das beleuchtete Bild über ein in axialer Richtung einstellbares Objektiv aufgenommen und einem Okular oder einer Kamera zugeleitet wird, wobei das Objektiv für mindestens zwei Bildschärfeneinstellungen durch elektromagnetische Beeinflussung einer als Anker dienenden Objektivfassung von einer Stellung in eine andere Stellung gegenüber dem distalen Ende eines Bildleiters verstellbar ist. Hierbei wird wenigstens eine der beiden Stellungen durch ein permanent anliegendes elektromagnetisches Feld hervorgerufen und die andere Stellung durch die Wirkung einer Feder.

EP 2 175 458 A2 offenbart einen Linearmotor für optische Systeme, beispielsweise Endoskope. Der Motor hat einen Stator mit zwei Permanentmagneten, die gleichsinnig gepolt und mit einem Rückschlusselement magnetisch miteinander verbunden sind. Eine Spule ist zwischen den Magneten angeordnet.

DE 37 17 872 A1 offenbart einen Linearmotor mit einer ersten Jochanordnung mit einem Jochpaar und einem dazwischen liegen den Spalt, einer zweiten Jochanordnung, die gleitend mit der ersten Jochanordnung verbunden ist, eine Permanentmagnetanordnung zur Erzeugung von durch die ersten und zweiten Jochanordnungen verlaufenden magnetischen Flüssen und eine Spulenanordnung an der zweiten Jochanordnung zur Erzeugung eines steuernden magnetischen Flusses zur Festlegung der relativen Lage der ersten und zweiten Jochanordnungen zueinander.

DE 1 253 407 B offenbart ein Endoskop mit einer distal ausstrahlenden Beleuchtungseinrichtung für den zu beobachtenden Körperhöhlenteil und einem Bildleiter, von dem das beleuchtete Bild über ein in axialer Richtung einstellbares Objektiv aufgenommen und einem Okular oder einer Kamera zugeleitet wird, wobei das Objektiv für mindestens zwei Bildschärfeneinstellungen durch elektromagnetische Beeinflussung einer als Anker dienenden Objektivfassung von einer Stellung in eine andere Stellung gegenüber dem distalen Ende eines Bildleiters verstellbar ist.

DE 200 00 397 U1 offenbart eine Magnetvorrichtung mit wechselbarem Magnetkreis mit einem Außengehäuse, dessen ringförmige Innenfläche mit Spule versehen ist, wobei ein Platz im Inneren des Außengehäuses reserviert ist, damit ein Eisenkern in seiner Axialrichtung verschoben werden kann. Hierbei wird mit einem Stromstoß dafür gesorgt, dass der Eisenkern von einer Position in eine andere Position verschoben wird.

Es ist Aufgabe der vorliegenden Erfindung, einen elektromagnetischen Aktuator anzugeben, mittels dem ein leistungsloses Halten des verschiebbaren Elements in definierten Positionen möglich ist, wobei das Verschieben des verschiebbaren Elements des Aktuators bei geringer Leistung ermöglicht sein soll.

Gelöst wird diese Aufgabe durch den Gegenstand des Anspruchs 1. Weiterbildungen der Erfindung sind Gegenstand der abhängigen Ansprüche.

Die Aufgabe wird durch einen elektromagnetischen Aktuator für ein chirurgisches oder medizinisches Instrument, insbesondere ein Endoskop, gelöst, wobei der Aktuator einen Stator und ein verschiebbares Element aufweist, das wenigstens teilweise ein paramagnetisches und/oder ferromagnetisches Material aufweist und durch Beaufschlagung mit einem elektromagnetischen Feld von einer ersten Position in eine zweite Position verschiebbar ist, wobei das verschiebbare Element in der ersten Position durch ein Permanentmagnetfeld gehalten wird oder ist und nach Verschieben in die zweite Position in der zweiten Position durch ein Permanentmagnetfeld gehalten wird oder ist, wobei zwei Anschläge vorgesehen sind, die die erste und die zweite Position definieren, wobei bei Anliegen des verschiebbaren Elements an einem Anschlag eine Kraft in Richtung der Anschläge auf das verschiebbare Element wirkt.

Durch Verwendung eines Permanentmagnetfeldes ist es möglich, das verschiebbare Element, insbesondere nacheinander, leistungslos sowohl in der ersten als auch der zweiten Position zu halten, so dass keine weitere Leistung in das System eingebracht werden muss. Der Stator umfasst zwei Permanentmagneten die gegensätzlich zueinander gepolt sind. Im Rahmen der Erfindung bedeutet gegensätzlich zueinander gepolt insbesondere, dass die zueinander angeordneten Pole der zwei Permanentmagnete sich abstoßen, also die gleichen Pole benachbart zueinander sind. Hierdurch ist es besonders einfach, ein leistungsloses Halten des verschiebbaren Elements in der ersten und/oder der zweiten Position zu ermöglichen. Das verschiebbare Element umfasst hierbei vorzugsweise keinen Permanentmagneten, sondern besteht ausschließlich aus einem paramagnetischen und/oder einem ferromagnetischen Material und gegebenenfalls zusätzlich aus einem nicht magnetischen Material, wobei das ferromagnetische Material aufgrund der größeren magnetfeldverstärkenden Wirkung bevorzugt ist. Zur Erzeugung des elektromagnetischen Feldes ist eine Spule vorgesehen, die zwischen den Permanentmagneten angeordnet ist. Durch diese Anordnung ist es möglich, auch mit einem relativ kleinen elektromagnetischen Feld das verschiebbare Element zu verschieben. Beim Verschieben bzw. Schalten des elektromagnetischen Aktuators wirken das Permanentmagnetfeld der beiden Permanentmagneten und das elektromagnetische Feld der Spule zusammen. Hierdurch ist es ermöglicht, dass die Permanentmagneten durch das elektromagnetische Feld nicht entmagnetisiert werden.

Erfindungsgemäß sind zwei Anschläge vorgesehen, die die erste und die zweite Position definieren. Durch die Anschläge kommt das verschiebbare Element in entsprechende Endpositionen oder Zwischenpositionen, über die das verschiebbare Element nicht hinausgelangen kann. Erfindungsgemäß wirkt beim Anliegen des verschiebbaren Elements an einen Anschlag eine Kraft in Richtung des Anschlags auf das verschiebbare Element. Hierbei wird vorzugsweise das verschiebbare Element in Richtung einer metastabilen Position gezogen, in die das verschiebbare Element allerdings aufgrund der Anschläge nicht ganz gelangen kann. Es wirkt insofern in den jeweiligen Positionen, also in der ersten Position, in dem Fall, in dem das verschiebbare Element in der ersten Position anliegt und auch in dem Fall, in dem das verschiebbare Element in der zweiten Position anliegt, eine magnetische Kraft in Richtung des jeweiligen Anschlags, so dass das verschiebbare Element definiert am Anschlag gehalten wird. Hierdurch ergibt sich eine sehr definierte Position.

Anstelle des Anschlags wäre es auch möglich, aber nicht beansprucht, keinen Anschlag vorzusehen und eine erste bzw. zweite Position im Bereich eines energetischen Minimums des Zusammenwirkens des Permanentmagnetfeldes durch die Permanentmagneten und des Materials des verschiebbaren Elements zu ermöglichen. Die Variante mit den Anschlägen ist allerdings aufgrund der definierten Positionen deutlich bevorzugt. Zwischen dem Permanentmagneten des Stators ist ein ferromagnetisches Material angeordnet. Eine besonders kleine Leistung für das elektromagnetische Feld ist ausreichend, um ein Verschieben des verschiebbaren Elements von einer ersten Position in eine zweite Position oder umgekehrt zu ermöglichen. Das ferromagnetische Material ist hierbei insbesondere Teil des Stators. Die Spule ist von den Permanentmagneten und dem ferromagnetischen Material des Stators, nach außen hin umschlossen. Durch die Anordnung eines paramagnetischen und/oder ferromagnetischen Materials im verschiebbaren Element als auch eines ferromagnetischen Materials im Stator, wird ein weichmagnetischer Rückschluss für die Spule erzeugt, wodurch schon bei kleinen Strömen durch die Spule hohe Magnetfelder und damit hohe Leistungsdichten erzielt werden können.

Vorzugsweise ist das verschiebbare Element in einem Rohr längsaxial verschiebbar gelagert. Die längsaxiale Verschiebung ist entlang der Längsachse des Rohrs. Vorzugsweise, aber nicht beansprucht, ist das Rohr zylinderförmig. Es wird vorzugsweise aber nicht beansprucht, ein um die Längsachse symmetrisches, insbesondere rotationssymmetrisches, Magnetfeld erzeugt. Hierdurch und insbesondere durch die Maßnahme, dass das verschiebbare Element, die Spule und die Permanentmagneten im Schnitt ringförmig sind, und zwar insbesondere im Schnitt quer zur Längsachse, wirken gleichmäßige Kräfte auf das verschiebbare Element, so dass ein Verschieben mit einer geringen Leistung möglich ist. Für den Verschiebevorgang des verschiebbaren Elements bzw. den Schaltvorgang von einer ersten Position in eine zweite Position oder umgekehrt, reicht ein kurzer elektrischer Schaltimpuls durch die Spule von weniger als 100 Millisekunden und weniger als 500 Milliampere.

Vorzugsweise ist ein chirurgisches oder medizinisches Instrument, insbesondere ein Endoskop, mit einem erfindungsgemäßen elektromagnetischen Aktuator versehen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische, dreidimensionale Schnittdarstellung durch einen Teil eines Endoskops mit einem erfindungsgemäßen Aktuator,
- Fig. 2: eine schematische Ausschnittsvergrößerung aus Fig. 1,
- Fig. 3: eine schematische Schnittdarstellung einer anderen Ausführungsform eines erfindungsgemäßen Aktuators,
- Fig. 4: eine schematische Schnittdarstellung der Ausführungsform aus Fig. 3 mit einer schematischen Flussdarstellung und
- Fig. 5: eine schematische Schnittdarstellung der Ausführungsform aus Fig. 3 mit einer schematischen Flussdarstellung.

In den folgenden Figuren sind jeweils gleiche oder gleichartige Elemente bzw. entsprechende Teile mit denselben Bezugsziffern versehen, so dass von einer entsprechenden erneuten Vorstellung abgesehen wird.

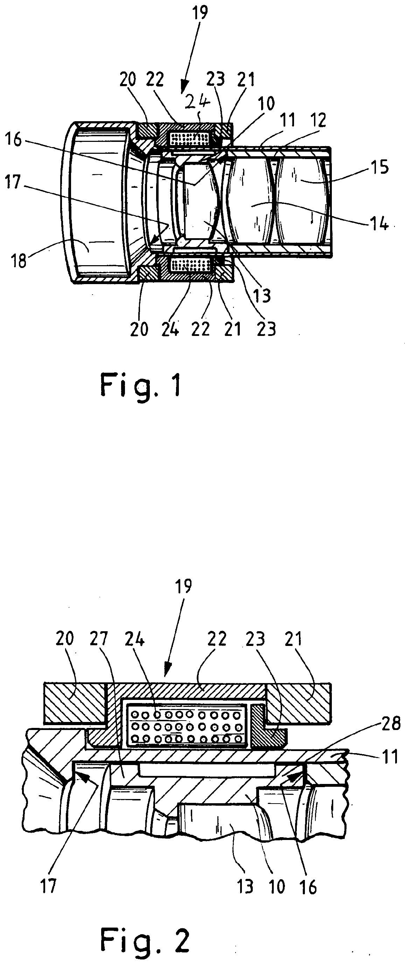

Fig. 1 zeigt eine schematische, dreidimensionale Schnittdarstellung durch einen Teil eines Endoskops mit einem erfindungsgemäßen Aktuator. Der Aktuator kann in einem nicht dargestellten Schaft eines Endoskops angeordnet sein. Der Schaft des Endoskops wäre in Fig. 1 koaxial um den Aktuator angeordnet, und zwar koaxial mit einem Durchmesser, der etwas größer ist als der Außendurchmesser des distalen Endes 18 des Gleitrohrs 11.

Das Gleitrohr 11, das aus einem Metall oder Kunststoff sein kann, wobei es hierbei darauf ankommt, dass dieses aus nichtmagnetischen Materialien besteht, dient als radiale Führung des verschiebbaren Elements 10. Das verschiebbare Element 10 kann beispielsweise eine Linse 13 aufweisen, die Bestandteil eines Objektivs ist, das außerdem noch Linsen 14 und 15 aufweist, die in einem fixierten Halteelement 12 eingebracht sind und entsprechend gehalten sind. Das fixierte Halteelement 12 ist in dem Gleitrohr 11 fixiert bzw. angebracht und definiert einen Anschlag 16. Der weitere Anschlag 17 zum distalen Ende wird auch durch das Gleitrohr 11 durch einen Kragen nach innen definiert. Bei diesem Ausführungsbeispiel gemäß Fig. 1 handelt es sich um einen rotationssymmetrischen Aufbau, bei dem ein axial verschiebbares Element 10 vorgesehen ist. Das verschiebbare Element 10 kann aus einer, wie in Fig. 1 dargestellten, proximalen Position in Fig. 1 nach links an den Anschlag 17 verschoben werden in eine distale Position. Das verschiebbare Element 10 ist als eine Art Hülse ausgebildet, die insbesondere aus einem weichmagnetischen Material, wie z.B. einem ferromagnetischen Material, besteht bzw. dieses Material aufweist.

Außer aus ferromagnetischem und/oder paramagnetischem Material kann das verschiebbare Element 10 noch eine Reibung vermindernde Beschichtung auf der Oberfläche aufweisen, die zur Innenwand des Gleitrohrs 11 angeordnet ist.

In Fig. 2 ist eine Ausschnittsvergrößerung aus Fig. 1 dargestellt, bei der die Form der jeweiligen Elemente deutlicher erkennbar ist. Das verschiebbare Element 10 weist einen distalen Polschuh 27 und einen proximalen Polschuh 28 auf. Diese wirken mit dem Magnetfeld und den Permanentmagneten 20 und 21, die als Ringe ausbildet sind und rotationssymmetrisch um die Längsachse des elektromagnetischen Aktuators angeordnet sind, zusammen. Zwischen den Permanentmagneten 20 und 21 sind ein erstes Zwischenteil 22 und ein zweites Zwischenteil 23 aus paramagnetischem oder ferromagnetischem Material vorgesehen, die auch mit Polschuhen oder als Polschuhe ausgebildet sind. Das erste Zwischenteil 22 und das zweite Zwischenteil 23 können auch einstückig sein, also ein einziges Zwischenteil bilden. Ferner ist eine Spule 24 vorgesehen, die nach außen hin durch das erste Zwischenteil 22 und das zweite Zwischenteil 23 umschlossen ist und nach innen bis auf die Unterbrechung durch das Gleitrohr 11 auch von paramagnetischem und/oder ferromagnetischem Material des verschiebbaren Elements 10 umgeben ist. Hierdurch wird eine sehr große Verstärkung des elektromagnetischen Feldes erzielt. Der Stator 19 des elektromagnetischen Aktuators besteht im Wesentlichen aus den beiden Permanentmagnetringen 20 und 21, den beiden Zwischenteilen 22 und 23 und der Spule 24.

Das Material, aus dem das verschiebbare Element 10 bestehen kann bzw. das dieses aufweist, kann beispielsweise St-37 oder C-45k sein. Die Außenkontur des verschiebbaren Elements stellt einen Doppelanker dar. Hierdurch entstehen zwei Polschuhe, nämlich ein distaler Polschuh 27 und ein proximaler Polschuh 28. Die Außenseiten der Polschuhe dienen darüber hinaus als Gleitflächen für die Gleitpaarung zwischen dem Gleitrohr 11 und dem verschiebbaren Element 10. Die Innenkontur des verschiebbaren Elements ist vorzugsweise achsensymmetrisch. Von der Symmetrie kann jedoch in gewissen Grenzen abgewichen werden, um beispielsweise eine Schulter zur Montage einer Linse 13 zu integrieren. Vorzugsweise ist das verschiebbare Element schwarz matt ausgeführt.

Der Stator 19 umfasst im Wesentlichen zwei gleichartige Permanentmagneten, die das gleiche Material bzw. die gleiche Magnet- und Magnetisierungsstärke und entsprechend die gleichen Dimensionen haben. Ferner ist eine Spule 24 vorgesehen sowie zwei ferromagnetische Bauteile bzw. Zwischenteile 22 und 23, die als magnetische Flussführung zur Verstärkung und Fokussierung von Magnetfeldern dienen. Die Zwischenteile 22 und 23 sind hufeisenförmig in einem Schnitt längsaxial durch den Stator und in einer polschuhartigen symmetrischen Ausführung realisiert. Sowohl das verschiebbare Element 10 als auch der Stator 19 sind vorzugsweise achsensymmetrisch aufgebaut. Die Permanentmagneten 20 und 21 sind gegensätzlich zueinander gepolt bzw. angestellt montiert.

Der elektromagnetische Aktuator kann in vier verschiedenen Zuständen vorliegen. Der erste Zustand ist der in Fig. 1 und 2 dargestellte Zustand, bei dem sich das verschiebbare Element 10 in der stabilen proximalen Position befindet. Hierbei wirkt die resultierende Kraft der Permanentmagneten auf das verschiebbare Element gegen den proximalen Anschlag 16. Ferner kann sich das verschiebbare Element in einer stabilen distalen Position befinden, die nicht in Fig. 1 und 2 dargestellt ist. Die resultierende Kraft der Permanentmagneten wirkt dann auf das verschiebbare Element 10 gegen den distalen Anschlag 17.

Der dritte Zustand ist der, dass der Aktuator das verschiebbare Element aus der distalen Position herausbewegt. Die resultierende Kraft der Spule und der Permanentmagneten bewegt das verschiebbare Element 10 dann in proximale Richtung. Umgekehrt ist der vierte Zustand definiert, in der der Aktuator das verschiebbare Element 10 aus der proximalen Position herausbewegt. Hierbei ist die resultierende Kraft der Spule und der Permanentmagneten so, dass das verschiebbare Element 10 in distale Richtung verschoben wird.

Die Funktionsweise wird im Folgenden näher erläutert.

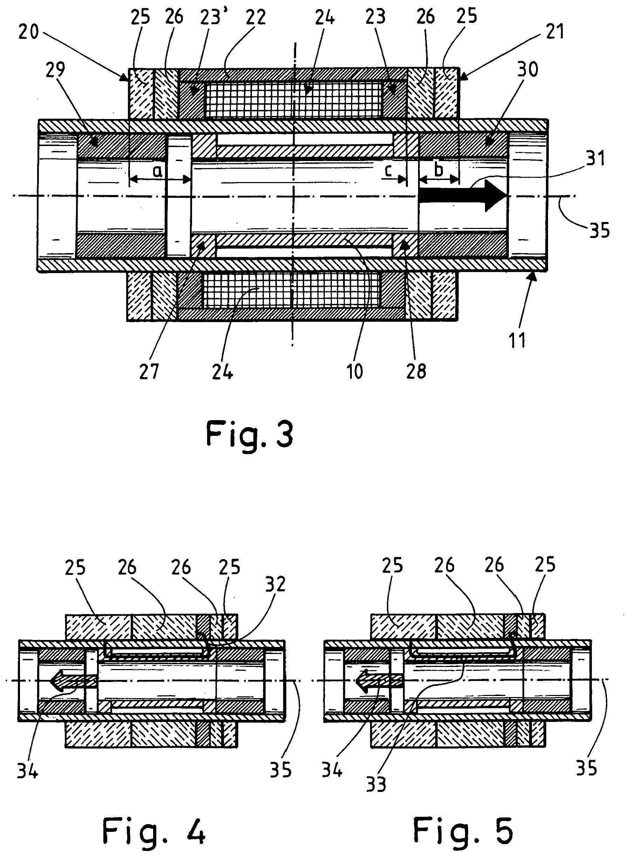

In den Fig. 3 bis 5 sind schematische Schnittdarstellungen durch einen elektromagnetischen Aktuator gezeigt, wobei die jeweiligen Elemente und Merkmale schematisch angedeutet sind. In Fig. 3 ist die Spule 24 stromlos, d.h. diese erzeugt kein Magnetfeld. Der Stator umfasst entsprechend wie in Fig. 1 und 2 aus einem ferromagnetischen Material bestehende Zwischenteile 22, 23 und 23', die im Schnitt hufeisenförmig ausgebildet sind. Die Zwischenteile 22, 23 und 23' können als ein gemeinsames Stück, also einstückig, gefertigt sein.

Mit 25 ist schematisch ein magnetischer Südpol dargestellt und mit 26 ein schematischer magnetischer Nordpol. Mit 22 ist ein erstes Zwischenteil bzw. Bauteil dargestellt und mit 23 sowie 23' jeweils ein zweites Zwischenteil bzw. Bauteil, die als Polschuhe ausgebildet sind. Entsprechend können auch die Elemente 10, 27 und 28, die die ferromagnetischen Teile des verschiebbaren Elements 10 darstellen sollen, gemeinsam einstückig sein. 27 bezeichnet den distalen Polschuh und 28 den proximalen Polschuh.

Die Haltekräfte des verschiebbaren Elements werden in diesem Fall nur von den beiden Permanentmagneten durch ein Permanentmagnetfeld erzeugt. Durch die angestellten Magnete 20 und 21 befindet sich an beiden Polschuhen 23 und 23' des Stators der gleiche magnetische Pol. Der magnetische Fluss ist bestrebt, den Weg des geringsten magnetischen Widerstandes zu gehen. Im Verhältnis zur Luft ist der magnetische Widerstand von dem verwendeten ferromagnetischen Material weitaus geringer, so dass das System insgesamt versucht, die Luftspalte zu minimieren. Dieses wird als Reluktanz bezeichnet. Hierdurch werden die Polschuhe, die vorzugsweise aus weichmagnetischem bzw. ferromagnetischem Material bestehen, in Überdeckung gebracht, wodurch eine Bewegung bzw. eine Kraft realisiert wird.

Um eine Haltekraft in proximale Richtung, wie in Fig. 3 durch die Kraft 31 angedeutet ist, an das proximale Anschlagelement 30 zu erreichen, sollte folgendes gegeben sein. Der proximale Polschuh 28 des verschiebbaren Elements 10 muss näher zum proximalen Ende des proximalen Permanentmagnets 21 positioniert sein als der distale Polschuh 27 des verschiebbaren Elements zum distalen Ende des distalen Permanentmagnets 20. Damit muss a größer als b sein. Zudem muss der proximale Polschuh 28 des verschiebbaren Elements 10 proximal über den proximalen Polschuh 23 des Ankers hinausragen. c muss also größer als Null sein. Wenn c = 0 wäre, wäre das System im magnetischen bzw. im energetischen Minimum. Dann wäre keine resultierende Kraft 31 mehr vorhanden. Eine entsprechende Kraft in Richtung des energetischen Minimums würde nur bei einer Verschiebung aus dieser Position heraus entstehen. Dieses führt zu einer nicht diskreten Positionierung, weswegen die Ausführungsform mit entsprechendem Anschlag bevorzugt ist.

Das verschiebbare Element 10 bildet für beide Magneten 20 und 21 den magnetischen Rückschluss, so dass der geringste magnetische Widerstand bzw. der energetisch günstigste Zustand des Systems über das verschiebbare Element 10 erreicht werden kann. Abhängig von der Position des verschiebbaren Elements, also auch abhängig von der Lage der Anschlagelemente 29 bzw. 30, können somit unterschiedliche Haltekräfte realisiert werden. In dem dargestellten Beispiel ist der elektromagnetische Aktuator so ausgelegt, dass die Position des verschiebbaren Elements 10 am Anschlag, also beispielsweise am proximalen Anschlagelement 30, nicht dem energetisch günstigsten Zustand entspricht. Dadurch wird der elektromagnetische Aktuator weiterhin versuchen, das verschiebbare Element in die Position des geringsten Widerstands zu ziehen, wodurch die resultierende Haltekraft (Reluktanz) hervorgeht.

Um nun das verschiebbare Element 10 von der proximalen Position in die distale Position zu verschieben, wird die Spule 24 bestromt. Hierdurch kann ein Gesamtmagnetfeld erzeugt werden, das eine Kraft in distale Richtung erzeugt, die größer ist als die Haltekraft in proximaler Richtung. Dieses ist in Fig. 4 und 5 dargestellt. Die Kraft in distale Richtung ist als Verschiebekraft 34 angegeben. Durch Bestromung der Spule 24 ergibt sich ein entsprechendes Magnetfeld in der Summation des Magnetfeldes des distalen Permanentmagnets 20 und der Spule, das schematisch durch einen magnetischen Nordpol 26 und einen magnetischen Südpol 25 auf der linken Seite der Fig. 4 und der Fig. 5 angedeutet ist. Im Idealfall erzeugt die Spule einen magnetischen Fluss, der dem des distalen Permanentmagnets 20 entspricht. Dadurch wird das Magnetfeld hin zum proximalen zweiten Zwischenteil 23 bzw. Statorpolschuh verstärkt. Der distale Permanentmagnet 20 und die Spule bilden, abstrahiert betrachtet, einen großen zusammenhängenden Magneten, der schematisch eine größere, idealerweise doppelte, Feldstärke als der proximale Permanentmagnet 21 aufweist. Hierdurch ergeben sich entsprechende magnetische Flüsse 32 und 33, die in den Fig. 4 und 5 dargestellt sind, und eine entsprechende Verschiebekraft 34 zum distalen Ende hin. Durch das Zusammenwirken der drei magnetischen Bauteile (beide Permanentmagnete 20 und 21 sowie der Spule 24) wird das verschiebbare Element 10 aus seiner proximalen Position zu seiner distalen Position bewegt.

Durch den dargestellten Aufbau ist es nicht notwendig, dass der magnetische Fluss der Spule den magnetischen Fluss eines Permanentmagnets vollständig auslöscht. Hierdurch wird die Gefahr, dass das Magnetfeld der Spule die Permanentmagnete entmagnetisiert, verringert. Durch das Umgeben der Spule durch ferromagnetisches Material ist ein sehr hoher Wirkungsgrad erzielbar. Dies minimiert den notwendigen Schaltstrom und damit eine möglicherweise auftretende Erwärmung, die bei einem Endoskop im distalen Bereich vermieden werden soll.

Der elektromagnetische Aktuator wird vorzugsweise bei Endoskopen verwendet, die ein optisches System aufweisen. Insbesondere kann mit dem elektromagnetischen Aktuator eine Linse verschoben werden, so dass diese längsaxial entlang der Längsachse 35 verschoben werden kann. Hierdurch ist eine Fokussierung oder eine Verschiebung der Brennweite des Objektivs ermöglicht. Anstelle oder zusätzlich zu der Linse kann auch ein Spiegel vorgesehen sein, mittels dessen die Blickrichtung eines Operateurs im distalen Bereich des Endoskops verändert werden kann. Durch die erfindungsgemäße Lösung ist ein geringer Bauaufwand mit geringem Platzbedarf realisierbar, so dass das Lumen, das beispielsweise für Linsen zur Verfügung steht, nur wenig reduziert wird, so dass sehr leuchtstarke Objektive und damit auch leuchtstarke Endoskope realisierbar sind.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

### Bezugszeichenliste

- 10: verschiebbares Element
- 11: Gleitrohr
- 12: fixiertes Halteelement
- 13: Linse
- 14: Linse
- 15: Linse
- 16: Anschlag
- 17: Anschlag
- 18: distales Ende
- 19: Stator
- 20: Permanentmagnet
- 21: Permanentmagnet
- 22: 1. Zwischenteil
- 23, 23': 2. Zwischenteil
- 24: Spule
- 25: magnetischer Südpol
- 26: magnetischer Nordpol
- 27: distaler Polschuh
- 28: proximaler Polschuh
- 29: distales Anschlagelement
- 30: proximales Anschlagelement
- 31: Kraft
- 32: magnetischer Fluss
- 33: magnetischer Fluss
- 34: Verschiebekraft
- 35: Längsachse
- a: Abstand
- b: Abstand
- c: Abstand

## Patentansprüche

1. Elektromagnetischer Aktuator für ein chirurgisches oder medizinisches Instrument, wobei der Aktuator einen Stator (19) und ein verschiebbares Element (10) aufweist, das wenigstens teilweise ein paramagnetisches und/oder ferromagnetisches Material aufweist und durch Beaufschlagung mit einem elektromagnetischen Feld von einer ersten Position in eine zweite Position verschiebbar ist, **dadurch gekennzeichnet, dass** das verschiebbare Element (10) in der ersten Position durch ein Permanentmagnetfeld gehalten wird oder ist und nach Verschieben in die zweite Position in der zweiten Position durch ein Permanentmagnetfeld gehalten wird oder ist, wobei zwei Anschläge (16, 17) vorgesehen sind, die die erste und die zweite Position definieren, wobei bei Anliegen des verschiebbaren Elements (10) an einem Anschlag (16, 17) eine Kraft (31) in Richtung der Anschläge (16, 17) auf das verschiebbare Element wirkt, wobei der Stator (19) zwei Permanentmagneten (20, 21) umfasst, die gegensätzlich zueinander gepolt sind, wobei eine Spule (24) zur Erzeugung des elektromagnetischen Feldes vorgesehen ist, wobei zwischen den Permanentmagneten (20, 21) des Stators (19) ein ferromagnetisches Material angeordnet ist, wobei die Spule (24) von den Permanentmagneten (20, 21) und dem ferromagnetischen Material (22, 23, 23') nach außen hin umschlossen ist, wobei das ferromagnetische Material im Längsaxial-Schnitt hufeisenförmig ausgebildet ist.

2. Elektromagnetischer Aktuator nach Anspruch 1, **dadurch gekennzeichnet, dass** die Spule (24) zwischen den Permanentmagneten (20, 21) angeordnet ist.

3. Elektromagnetischer Aktuator nach Anspruch 1 oder 2, **dadurch gekennzeichnet, dass** das verschiebbare Element (10) in einem Rohr (11) längsaxial verschiebbar gelagert ist.

4. Elektromagnetischer Aktuator nach einem der Ansprüche 1 bis 3, **dadurch gekennzeichnet, dass** das verschiebbare Element (10), die Spule (24) und die Permanentmagneten (20, 21) im Schnitt ringförmig sind.

5. Chirurgisches oder medizinisches Instrument, insbesondere Endoskop, mit einem elektromagnetischen Aktuator nach einem der Ansprüche 1 bis 4.

## Claims

1. An electromagnetic actuator for a surgical or medical instrument, wherein the actuator has a stator (19) and a shiftable element (10) that has at least partially a paramagnetic and/or ferromagnetic material and can be shifted from a first position into a second position by applying an electromagnetic field, **characterized in that** the shiftable element (10) is or will be held in the first position by a permanent-magnetic field and after shifting into the second position is or will be held in the second position by a permanent-magnetic field, wherein two stops (16, 17) are provided that define the first and the second position, wherein when the shiftable element (10) abuts a stop (16, 17) a force (31) acts on the shiftable element in the direction of the stops (16, 17), wherein the stator (19) comprises two permanent magnets (20, 21) that are polarized opposite to each other, wherein a coil (24) is provided for generating the electromagnetic field, wherein a ferromagnetic material is arranged between the permanent magnets (20, 21) of the stator (19), wherein the coil (24) is surrounded to the outside by the permanent magnets (20, 21) and the ferromagnetic material (22, 23, 23'), wherein the ferromagnetic material is designed horseshoe-shaped in the longitudinal axial section.

2. The electromagnetic actuator according to claim 1, **characterized in that** the coil (24) is arranged between the permanent magnets (20, 21).

3. The electromagnetic actuator according to claim 1 or 2, **characterized in that** the shiftable element (10) is mounted shiftably in a longitudinal axial manner in a tube (11).

4. The electromagnetic actuator according to one of claims 1 to 3, **characterized in that** the shiftable element (10), the coil (24) and the permanent magnets (20, 21) are sectionally ring-shaped.

5. A surgical or medical instrument, in particular an endoscope, with an electromagnetic actuator according to one of claims 1 to 4.

## Revendications

1. Actionneur électromagnétique pour un instrument chirurgical ou médical, dans lequel l'actionneur comporte un stator (19) et un élément apte à être déplacé (10) qui comporte au moins en partie un matériau paramagnétique et/ou ferromagnétique et qui peut être déplacé d'une première position à une deuxième position en appliquant un champ électromagnétique, **caractérisé en ce que** l'élément apte à être déplacé (10) est ou sera maintenu dans la première position par un champ magnétique permanent et est ou sera maintenu dans la deuxième position par un champ magnétique permanent après déplacement dans la deuxième position, deux butées (16, 17) étant prévues, qui définissent la première et la deuxième position, une force (31) agissant en direction des butées (16, 17) sur l'élément apte à être déplacé, lors d'un contact de l'élément apte à être déplacé (10) contre une butée (16, 17), le stator (19) comprenant deux aimants permanents (20, 21) qui ont des polarités opposées, une bobine (24) étant prévue pour générer le champ électromagnétique, un matériau ferromagnétique étant agencé entre les aimants permanents (20, 21) du stator (19), la bobine (24) étant enfermée vers l'extérieur par les aimants permanents (20, 21) et le matériau ferromagnétique (22, 23, 23'), le matériau ferromagnétique présentant une forme de fer à cheval en coupe longitudinale axiale.

2. Actionneur électromagnétique selon la revendication 1, **caractérisé en ce que** la bobine (24) est agencée entre les aimants permanents (20, 21).

3. Actionneur électromagnétique selon la revendication 1 ou la revendication 2, **caractérisé en ce que** l'élément apte à être déplacé (10) est monté de manière à pouvoir être déplacé longitudinalement et axialement dans un tube (11).

4. Actionneur électromagnétique selon l'une des revendications 1 à 3, **caractérisé en ce que** l'élément apte à être déplacé (10), la bobine (24) et les aimants permanents (20, 21) sont de forme annulaire en section.

5. Instrument chirurgical ou médical, en particulier un endoscope, comportant un actionneur électromagnétique selon l'une des revendications 1 à 4.
